# EUROPEAN PATENT APPLICATION

(11) **EP 1 577 280 A1**
(43) Date of publication of application: **21.09.2005**
(21) Application number: 03814536.3
(22) Date of filing: 07.11.2003
(51) Int. Cl.: C07B 59/00, C07C 29/00, C07C 31/02, C07C 35/08, C07C 35/29, C07C 35/37, C07C 45/00, C07C 49/04, C07C 49/08, C07C 49/433, C07C 49/453, C07C 51/00, C07C 53/10, C07C 53/124, C07C 57/04

(54) **METHOD OF DEUTERIZATION**

(30) Priority: 27.12.2002 JP 2002378932
(71) Applicant: Wako Pure Chemical Industries, Ltd., Osaka 540-8605 (JP)
(72) Inventor: ITO, Nobuhiro, Kawagoe-shi, Saitama 350-1101 (JP); MAESAWA, Tsuneaki, Kawagoe-shi, Saitama 350-1101 (JP); MUTO, Kazushige, Kawagoe-shi, Saitama 350-1101 (JP); HIROTA, Kosaku, Gifu-shi, Gifu 502-0003 (JP); SAJIKI, Hironao, Gifu-shi, Gifu 502-0823 (JP)
(74) Representative: Winter, Brandl,&Fürniss, Hübner Röss, Kaiser, Polte Partnerschaft Patent- und Rechtsanwaltskanzlei
(86) International application number: PCT/JP2003/014182
(87) International publication number: WO 2004/060831

(57) **Abstract**

The present invention relates to a method for deuteration of a compound represented by the general formula [1]:

R¹―X―R² [1]

wherein, R¹ represents an alkyl group or an aralkyl group, which may have a carbon-carbon double bond and/or triple bond; R² represents an alkyl group which may have a carbon-carbon double bond and/or triple bond, an aryl group, an aralkyl group, an alkoxy group, an aryloxy group or a hydroxyl group; X represents a carbonyl group or a hydroxylmethylene group; R¹ and R² may form an alicyclic ring together with a carbon atom contained in X; provided that R² represents an alkyl group which may have a carbon-carbon double bond and/or triple bond, an aryl group or an aralkyl group when X is a hydroxylmethylene group,
comprising reacting the compound represented by the general formula [1] with a heavy hydrogen source in the co-presence of an activated catalyst selected from a palladium catalyst, a platinum catalyst, a rhodium catalyst, a ruthenium catalyst, a nickel catalyst and a cobalt catalyst. The method of the present invention can significantly improve working environment because the deuteration, which has been conventionally carried out under severe conditions such as basic condition, can be carried out under neutral condition. Further, even when the compound represented by the general formula [1] is one having a carbon-carbon double bond or triple bond, the method for deuteration of the present invention enables to efficiently carry out objective deuteration without reduction of said double bond or triple bond.

## Description

### TECHNICAL FIELD

The present invention relates to a method for deuteration of a compound, using an activated catalyst.

### BACKGROUND OF THE INVENTION

A compound having a heavy hydrogen (deuterium and tritium) is said to be useful in various purposes. For example, a deuterated compound is very useful in clarification of reaction mechanism and substance metabolism and used widely as a labeled compound. Said compound is also known to be useful as drugs, pesticides, organic EL materials, and the like due to change in stability and property itself by isotope effect thereof. A compound having tritium is also said to be useful as a labeled compound in animal tests and the like to survey absorption, distribution, concentration in blood, excretion, metabolism and the like of drugs, etc. Therefore, research on a compound having a heavy hydrogen (deuterium and tritium) has been increasing also in these fields.

Various methods for obtaining these compounds having a heavy hydrogen have conventionally been used, however, among others, there were many problems to be solved in deuteration technology of a compound having a carbonyl group or a hydroxyl group, and it was difficult to efficiently and industrially obtain a deuterated compound.

Conventional technology includes, for example, 1) a method for deuteration of a carboxylic acid under basic condition using heavy hydrogen peroxide (see USP 3849458), 2) a method for deuteration of an alcohol or a carboxylic acid using an iridium complex as a catalyst and heavy water as a heavy hydrogen source (see J. Am. Chem. Soc. Vol.124, No. 10, 2092 (2002)), 3) a method for deuteration of a fatty acid using a palladium carbon as a catalyst and only heavy hydrogen gas as a heavy hydrogen source (see LIPIDS, Vol. 9, No. 11, 913 (1974), 4) a method for deuteration of acrylic acid, methyl acrylate, methacrylic acid or methyl methacrylate using a metal selected from the metals belonging to the 8th group metals as a catalyst and heavy water or heavy water + heavy hydrogen gas as a heavy hydrogen source (see JP-B-5-19536, JP-A-61-277648 and JP-A-61-275241) and 5) a method for deuteration of acrylic acid, methyl methacrylate, and the like using a catalyst not activated with hydrogen and heavy water as a heavy hydrogen source (see JP-A-63-198638).

However, each of these methods has problems as described below.
1) A method for deuteration of a carboxylic acid under basic condition using heavy hydrogen peroxide has a problem that this method cannot deuterate a compound labile to decomposition by heavy hydrogen peroxide or under basic condition, and further, complicated purification processes are required in isolation of thus deuterated compound, because reaction solution is not neutral, even if a compound not labile to decomposition under acidic or basic condition is used as a substrate.
2) A method for deuteration of an alcohol compound or a carboxylic acid using an iridium complex as a catalyst and heavy water as a heavy hydrogen source has a problem that deuteration ratio of a hydrogen atom becomes higher as the hydrogen atom is located at a more distant position from the carbon atom to which a hydroxyl group in an alcohol compound is bonded, and deuteration ratio of the hydrogen atom near the hydroxyl group becomes extremely low. Furthermore, an iridium complex to be used as a catalyst is difficult to manufacture or purchase because the compound itself is unstable.
3) A method for deuteration of a fatty acid using a palladium carbon as a catalyst and heavy hydrogen gas generated by electrolysis of KOD + D₂O as a heavy hydrogen source is not adequate for practical use due to requirement of a special apparatus for production of heavy hydrogen gas and very complicated operation thereof. Further, such a method using heavy hydrogen gas as a heavy hydrogen source can hardly deuterate a compound such as an unsaturated fatty acid having an unsaturated bond, which is reduced by hydrogenation.
4) A method for deuteration of acrylic acid, methyl acrylate, methacrylic acid or methyl methacrylate using a metal selected from the metals belonging to the 8th group metals as a catalyst and heavy water or heavy water + heavy hydrogen gas as a heavy hydrogen source has the following problems. Namely, when only heavy water is used as a heavy hydrogen source, deuteration ratio is low because a non-activated catalyst is used. On the other hand, when heavy water + heavy hydrogen gas are used as a heavy hydrogen source, hydrogenation (catalytic reduction) of a carbon-carbon double bond moiety of acrylic acid, methyl acrylate, methacrylic acid or methyl methacrylate as a reactive substrate easily occurs with heavy hydrogen gas as well as deuteration, and it is impossible to deuterate the compound leaving said bond unchanged.
5) A method, for deuteration of acrylic acid or methyl methacrylate using a catalyst not activated with hydrogen and heavy water as a heavy hydrogen source has a problem of low deuteration ratio due to use of a non-activated catalyst as a catalyst.

In view of the above situation, development of a method is needed for deuteration of a carbonyl compound or a secondary alcohol compound efficiently and industrially irrespective of kinds of a substituent and presence or non-presence of a double bond and a triple bond.

### SUMMARY OF THE INVENTION

The present invention relates to a method for deuteration of a compound represented by the general formula [1]:

R¹-X-R² [1]

wherein, R¹ represents an alkyl group or an aralkyl group, which may have a carbon-carbon double bond and/or a triple bond; R² represents an alkyl group which may have a carbon-carbon double bond and/or a triple bond, an aryl group, an aralkyl group, an alkoxy group, an aryloxy group or a hydroxyl group; X represents a carbonyl group or a hydroxylmethylene group; R¹ and R² may form an alicyclic ring together with a carbon atom contained in X; provided that R² represents an alkyl group which may have a carbon-carbon double bond and/or a triple bond when X is a hydroxylmethylene group, an aryl group or an aralkyl group,
which comprising reacting the compound represented by the general formula [1] with a heavy hydrogen source in the co-presence of an activated catalyst selected from a palladium catalyst, a platinum catalyst, a rhodium catalyst, a ruthenium catalyst, a nickel catalyst and a cobalt catalyst.

Further, the present invention also relates to a deuterated tricyclo[5.2.1.0^{2,6}]decan-8-ol wherein a deuteration ratio thereof is 60% or more.

### BEST MODE FOR CARRYING OUT THE INVENTION

In a present invention, a heavy hydrogen means deuterium (D) and tritium (T) and deuteration means substitution with deuterium and tritium. Further, in the present specification, deuteration ratio means a ratio of an amount of hydrogen atoms substituted by heavy hydrogen atom to the total amount of hydrogen atoms in a compound represented by the general formula [1].

In a method for deuteration of the present invention, the alkyl group of an alkyl group which may have a carbon-carbon double bond and/or triple bond, represented by R¹ and R² of a compound represented by the general formula [1] may be straight chained, branched or cyclic, and includes one generally having 1 to 20, preferably 1 to 15, more preferably 1 to 10 and further more preferably 1 to 6 carbon atoms, which is specifically exemplified by, for example, a methyl group, an ethyl group, a n-propyl group, an isopropyl group, a n-butyl group, an isobutyl group, a sec-butyl group, a tert-butyl group, a n-pentyl group, an isopentyl group, a sec-pentyl group, a tert-pentyl group, a neopentyl group, a n-hexyl group, an isohexyl group, a 3-methylpentyl group, a 2-methylpentyl group, a 1,2-dimethylbutyl group, a n-heptyl group, an isoheptyl group, a sec-heptyl group, a n-octyl group, an isooctyl group, a sec-octyl group, a n-nonyl group, a n-decyl group, a n-undecyl group, a n-dodecyl group, a n-tridecyl group, a n-tetradecyl group, a n-pentadecyl group, a n-hexadecyl group, a n-heptadecyl group, a n-octadecyl group, a n-nonadecyl group, a n-icosyl group, a cyclopropyl group, a cyclopentyl group, a cyclohexyl group, a cycloheptyl group, a cyclooctyl group, a cyclononyl group, a cyclodecyl group, a cycloundecyl group, a cyclododecyl group, a cyclotridecyl group, a cyclotetradecyl group, a cyclopentadecyl group, a cyclohexadecyl group, a cycloheptadecyl group, a cyclooctadecyl group, a cyclononadecyl group and a cycloicosyl group.

The alkyl group having a carbon-carbon double bond or triple bond includes one containing at least one double bond or triple bond in a chain of an alkyl group having not less than 2 carbon atoms among the above alkyl groups, and the alkyl group having a carbon-carbon double bond and triple bond includes one containing each at least one double bond and triple bond, in a chain of an alkyl group having not less than 4 carbon atoms among the above alkyl groups, and the specific examples of an alkyl group having such a carbon-carbon double bond and/or a triple bond include, for example, alkyl groups having only a carbon-carbon double bond such as a vinyl group, an allyl group, a 1-propenyl group, an isopropenyl group, a 3-butenyl group, a 2-butenyl group, a 1-butenyl group, a 1,3-butadienyl group, a 4-pentenyl group, a 3-pentenyl group, a 2-pentenyl group, a 1-pentenyl group, a 1,3-pentadienyl group, a 2,4-pentadienyl group, a 1,1-dimethyl-2-propenyl group, a 1-ethyl-2-propenyl group, a 1,2-dimethyl-1-propenyl group, a 1-methyl-1-butenyl group, a 5-hexenyl group, a 4-hexenyl group, a 2-hexenyl group, a 1-hexenyl group, a 1-methyl-1-hexenyl group, a 2-methyl-2-hexenyl group, a 3-methyl-1,3-hexadienyl group, a 1-heptenyl group, a 2-octenyl group, a 3-nonenyl group, a 4-decenyl group, a 1-dodecenyl group, a 1-tetradecenyl group, a 1-hexadecenyl group, a 1-octadecenyl group, a 1-icosenyl group, a 1-cyclopropenyl group, a 2-cyclopentenyl group, a 2,4-cyclopentadienyl group, a 1-cyclohexenyl group, a 2-cyclohexenyl group, a 3-cyclohexenyl group, a 2-cycloheptenyl group, a 2-cyclononenyl group, a 3-cyclodecenyl group, a 2-cyclotridecenyl group, a 1-cyclohexadecenyl group, a 1-cyclooctadecenyl group and a 1-cycloicosenyl group; alkyl groups having only a carbon-carbon triple bond such as an ethynyl group, a 2-propynyl group, a 1-propynyl group, a 2-pentynyl group, a 2-nonyl-3-butynyl group, a cyclohexyl-3-ynyl-group, a 4-octynyl group and a 1-methyldecyl-5-ynyl group; alkyl groups having both of a carbon-carbon double bond and triple bond such as a 1-buten-3-ynyl group, a 2-penten-4-ynyl group, a 5-(3-pentenyl)-3,6,8-decatrien-1-ynyl group, a 6-(1,3-pentadienyl)-2,4,7-dodecatrien-9-ynyl group and a 6-(1-penten-3-ynyl)-2,4,7,9-undecatetraenyl group.

The aralkyl group represented by R¹ and R² may be straight chained, branched or cyclic, and includes the above alkyl groups substituted with the above aryl groups, one having generally 7 to 34, preferably 7 to 20 and more preferably 7 to 15 carbon atoms, which is specifically exemplified by, for example, a benzyl group, a phenylethyl group, a phenylpropyl group, a phenylbutyl group, a phenylpentyl group, a phenylhexyl group, a phenylheptyl group, a phenyloctyl group, a phenylnonyl group, a phenyldecyl group, a phenylundecyl group, a phenyldodecyl group, a phenyltridecyl group, a phenyltetradecyl group, a phenylpentadecyl group, a phenylhexadecyl group, a phenylheptadecyl group, a phenyloctadecyl group, a phenylnonadecyl group, a phenylicosyl group, a naphthylethyl group, a naphthylpropyl group, a naphthylbutyl group, a naphthylpentyl group, a naphthylhexyl group, a naphthylheptyl group, a naphthyloctyl group, a naphthylnonyl group, a naphthyldecyl group, a naphthylundecyl group, a naphthyldodecyl group, a naphthyltridecyl group, a naphthyltetradecyl group, a naphthylpentadecyl group, a naphthylhexadecyl group, a naphthylheptadecyl group, a naphthyloctadecyl group, a naphthylnonadecyl group, a naphthylicosyl group, an anthrylethyl group, an anthrylpropyl group, an anthrylbutyl group, an anthrylpentyl group, an anthrylhexyl group, an anthrylheptyl group, an anthryloctyl group, an anthrylnonyl group, an anthryldecyl group, an anthrylundecyl group, an anthryldodecyl group, an anthryltridecyl group, an anthryltetradecyl group, an anthrylpentadecyl group, an anthrylhexadecyl group, an anthrylheptadecyl group, an anthryloctadecyl group, an anthrylnonadecyl group, an anthrylicosyl group, a phenanthrylethyl group, a phenanthrylpropyl group, a phenanthrylbutyl group, a phenanthrylpentyl group, a phenanthrylhexyl group, a phenanthrylheptyl group, a phenanthryloctyl group, a phenanthrylnonyl group, a phenanthryldecyl group, a phenanthrylundecyl group, a phenanthryldodecyl group, a phenanthryltridecyl group, a phenanthryltetradecyl group, a phenanthrylpentadecyl group, a phenanthrylhexadecyl group, a phenanthrylheptadecyl group, a phenanthryloctadecyl group, a phenanthrylnonadecyl group and a phenanthrylicosyl group.

The aryl group represented by R² includes one generally having 6 to 14, preferably 6 to 10 carbon atoms, which is specifically exemplified by, for example, a phenyl group, a naphthyl group and an anthryl group.

The alkoxy group represented by R² may be straight chained, branched or cyclic, and includes one generally having 1 to 20, preferably 1 to 15, more preferably 1 to 10 and further more preferably 1 to 6 carbon atoms, which is specifically exemplified by, for example, a methoxy group, an ethoxy group, a propoxy group, an isopropoxy group, a butoxy group, an isobutoxy group, a sec-butoxy group, a tert-butoxy group, a pentyloxy group, a neopentyloxy group, a hexyloxy group, an isohexyloxy group, a tert-hexyloxy group, a heptyloxy group, an octyloxy group, a nonyloxy group, a decyloxy group, an undecyloxy group, a tetradecyloxy group, a hexadecyloxy group, a heptadecyloxy group, a nonadecyloxy group, an icosyloxy group, a cyclohexyloxy group, a cyclooctyloxy group; a cyclodecyloxy group and a cyclononadecyloxy group.

The aryloxy group represented by R² includes one generally having 6 to 14, preferably 6 to 10 carbon atoms, which is specifically exemplified by a phenoxy group, a naphthyloxy group and an anthryloxy group.

The hydroxyl group represented by R² also includes one, wherein the hydrogen atom thereof is replaced by an alkali metal atom such as sodium, potassium and lithium.

The alicyclic ring, which is formed by R¹ and R² together with a carbon atom contained in X, may be a monocyclic ring or a polycyclic ring, and includes one having generally 3 to 15, preferably 5 to 10, more preferably 6 to 8 carbon atoms, which is specifically exemplified by, for example, a saturated monocyclic ring such as a cyclopropane ring, a cyclobutane ring, a cyclopentane ring, a cyclohexane ring, a cycloheptane ring, a cyclooctane ring, a cyclononane ring, a cyclodecane ring, a cycloundecane ring, a cyclododecane ring, a cyclotridecane ring, a cyclotetradecane ring and a cyclopentadecane ring; an unsaturated monocyclic ring such as a cyclobutenyl ring, a cyclopentenyl group, a cyclohexenyl group, a cycloheptenyl group, a cyclooctenyl group and a cyclononenyl group; a saturated or unsaturated polycyclic ring such as a tricyclodecane ring, a dicyclopentadiene group, a perhydronaphthalene ring, a perhydroanthracene ring, a norbomane ring, norpinane ring, a norcarane ring and an adamantane ring.

In the compound represented by the general formula [1] of the present invention, the alkyl group and the aralkyl group, which may have a carbon-carbon double bond and/or triple bond, represented by R¹ and R², and the aryl group, the alkoxy group and the aryloxy group, represented by R² may further have generally 1 to 5, preferably 1 to 3 various substituents. The substituent includes, for example, an alkyl group, an aryl group, an aralkyl group, an alkoxy group, an aryloxy group, an alkoxycarbonyl group, an aryloxycarbonyl group, an acyl group, a carboxyl group, an aldehyde group, a hydroxyl group, an amino group, an aminoalkyl group, a cyano group, a carbamoyl group and an alkylcarbamoyl group, which may have a carbon-carbon double bond and/or triple bond.

Specific examples of the alkyl group, the aralkyl group, the aryl group, the alkoxy group, the aryloxy group, which may have a carbon-carbon double bond and/or a triple bond and the hydroxyl group, as the above substituent of the group represented by R¹ and/or R² include the same one as those represented by R¹ and/or R².

Further, specific examples of the alkoxycarbonyl group and the aryloxycarbonyl group as the substituent of the group represented by R¹ and/or R² include one, wherein a carbonyl group is bonded to the oxygen atom of the above specific examples of the alkoxy group and the aryl group represented by R¹ and/or R².

The acyl group as a substituent of the group represented by R¹ and/or R² includes one having generally 2 to 20, preferably 2 to 10, more preferably 2 to 4 carbon atoms, which is specifically exemplified by, for example, an acyl group derived from saturated aliphatic monocarboxylic acids such as an acetyl group, a propionyl group, a butyryl group, an isobutyryl group, a valeryl group, an isovaleryl group, a pivaloyl group, a lauroyl group, a myristoyl group, a palmitoyl group and a stearoyl group; an acyl group derived from unsaturated aliphatic monocarboxylic acids such as an acryloyl group, a propioloyl group, a methacryoyl group, a crotonoyl group and an oleoyl group; an acyl group derived from aromatic monocarboxylic acids such as a benzoyl group and a naphthoyl group.

The carboxyl group as a substituent of the group represented by R¹ and/or R² also includes one, wherein the hydrogen atom thereof is replaced by an alkali metal atom such as sodium, potassium and lithium.

The amino group as a substituent of the group represented by R¹ and/or R² may be one, wherein 1 or 2 hydrogen atoms thereof are replaced by an alkyl group having generally 1 to 6, preferably 1 to 4 carbon atoms, which is straight chained, branched or cyclic.

The aminoalkyl group as a substituent of the group represented by R¹ and/or R² includes one, wherein at least one hydrogen atom of the alkyl group represented by R¹ and/or R² is replaced by the above amino group.

The alkylcarbamoyl group as a substituent of the group represented by R¹ and/or R² includes one, wherein 1 or 2 hydrogen atoms of the carbamoyl group are each independently replaced by the above alkyl group, which is specifically exemplified by, for example; a methylcarbamoyl group, an ethylcarbamoyl group, a n-propylcarbamoyl group, an isopropylcarbamoyl group, a n-butylcarbamoyl group, an isobutylcarbamoyl group, a tert-butylcarbamoyl group, a pentylcarbamoyl group, a hexylcarbamoyl group, a heptylcarbamoyl group, an octylcarbamoyl group, a nonylcarbamoyl group, a decylcarbamoyl group, a dodecylcarbamoyl group, a tetradecylcarbamoyl group, a pentadecylcarbamoyl group, a hexadecylcarbamoyl group, a heptadecylcarbamoyl group, a nonadecylcarbamoyl group, an icosylcarbamoyl group, a cyclopentylcarbamoyl group, a cyclohexylcarbamoyl group, a cycloheptylcarbamoyl group, dimethylcarbamoyl group, an ethylmethylcarbamoyl group, diethylcarbamoyl group, a methylpropylcarbamoyl group, dipropylcarbamoyl group, an ethylhexylcarbamoyl group, a dibutylcarbamoyl group, a heptylmethylcarbamoyl group, a methyloctylcarbamoyl group, a decylmethylcarbamoyl group, a dodecylethylcarbamoyl group, a methylpentadecylcarbamoyl group, an ethyloctadecylcarbamoyl group, a cyclopentylmethylcarbamoyl group, a cyclohexylmethylcarbamoyl group, a cyclohexylethylcarbamoyl group, a cyclohexylpropylcarbamoyl group, a cyclohexylbutylcarbamoyl group and a dicyclohexylcarbamoyl group.

Among the compounds represented by the general formula [1], deuteration of the compound having a substituent such as an alkoxycarbonyl group, an aryloxycarbonyl group and a cyano group, which is labile to decomposition under acidic or basic condition, can efficiently provide a desired deuterated compound without decomposition of these substituents by the use of the method of the present invention.

In a method for deuteration of the present invention, the heavy hydrogen source to be reacted with the above compound represented by the general formula [1] includes, for example, heavy hydrogen gas (D₂, T₂) and a deuterated solvent. As a heavy hydrogen source for deuteration of the compound represented by the general formula [1], a deuterated solvent is particularly preferable when X is a carbonyl group, and also a deuterated solvent is preferable when X is a hydroxylmethylene group.

Specific examples of the deuterated solvent as a heavy hydrogen source include, in the case where heavy hydrogen is deuterium, for example, deuterium oxide (D₂O); deuterated alcohols such as deuterated methanol, deuterated ethanol, deuterated isopropanol, deuterated butanol, deuterated tert-butanol, deuterated pentanol, deuterated hexanol, deuterated heptanol, deuterated octanol, deuterated nonanol, deuterated decanol, deuterated undecanol and deuterated dodecanol; deuterated carboxylic acids such as deuterated formic acid, deuterated acetic acid, deuterated propionic acid, deuterated butyric acid, deuterated isobutyric acid, deuterated valeric acid, deuterated isovaleric acid and deuterated pivalic acid; deuterated ketones such as deuterated acetone, deuterated methylethylketone, deuterated methylisobutyl ketone, deuterated diethylketone, deuterated dipropylketone, deuterated diisopropylketone and deuterated dibutylketone; and organic solvents such as deuterated dimethylsulfoxide, and among others, deuterium oxide and deuterated alcohols are preferable, and deuterium oxide and deuterated methanol are more preferable. In view of environmental aspect and workability, deuterium oxide is preferable. In the case where a heavy hydrogen is tritium, specific examples of the deuterated solvent as a heavy hydrogen source include, for example, tritium oxide (T₂O), etc.

The deuterated solvent may be one, wherein at least one hydrogen atom in the molecule is deuterated, and for example, deuterated alcohols wherein at least a hydrogen atom in a hydroxyl group is deuterated, or deuterated carboxylic acids wherein at least a hydrogen atom in a carboxyl group is deuterated, can be used in a method for deuteration of the present invention, and among others, a solvent wherein all hydrogen atoms in the molecule are deuterated is particularly preferable.

As an amount of heavy hydrogen source to be used is increasing, deuteration of the present invention tends to proceed further, however, in view of cost, the amount of a heavy hydrogen atom contained in heavy hydrogen source to be used is such level, as lower limit, preferably in the order of, equimolar, 10 molar times, 20 molar times, 30 molar times and 40 molar times, and as upper limit, preferably in the order or, 250 molar times and 150 molar times, relative to hydrogen atoms deuteratable in the compound represented by the general formula [1] as a reactive substrate.

In a method for deuteration of the present invention, a reaction solvent may be used, if necessary. In the case where a reactive substrate is liquid, use of a reaction solvent is not necessary, even if heavy hydrogen gas is used as a heavy hydrogen source, and in the case where a reactive substrate is solid, use of a reaction solvent is not required, when a deuterated solvent is used as a heavy hydrogen source, however, use of a suitable reaction solvent is necessary when a reactive substrate is solid and heavy hydrogen source is heavy hydrogen gas.

In the case where a compound having a carbon-carbon double bond or a carbon-carbon triple bond is deuterated, a deuterated solvent is preferably used as a heavy hydrogen source because these groups are reduced by so-called hydrogenation in contact with hydrogen gas or heavy hydrogen gas in the presence of a catalyst.

The reaction solvent to be used if necessary is preferably one not deuterated by heavy hydrogen gas used as heavy hydrogen source, or such one as even when deuterated by heavy hydrogen gas, said deuterated reaction solvent can be used as it is as heavy hydrogen source for deuteration of the present invention. A reaction solvent, which hardly dissolves a substrate, can be used, because a reaction system of deuteration of the present invention may be in suspension state, however, one, which easily dissolves a substrate, is preferable.

Specific examples of the reaction solvent to be used, if necessary, include organic solvents, which is not deuteratable by heavy hydrogen gas, such as ethers including dimethyl ether, diethyl ether, diisopropyl ether, ethylmethyl ether, tert-butylmethyl ether, 1,2-dimethoxyethane, oxirane, 1,4-dioxane, dihydropyran and tetrahydrofuran; aliphatic hydrocarbons including hexane, heptane, octane, nonane, decane and cyclohexane; organic solvents, which can be usable as heavy hydrogen source of the present invention even if deuterated by heavy hydrogen gas, such as alcohols including methanol, ethanol, isopropanol, butanol, tert-butanol, pentanol, hexanol, heptanol, octanol, nonanol, decanol, undecanol and dodecanol, carboxylic acids including formic acid, acetic acid, propionic acid, butyric acid, isobutyric acid, valeric acid, isovaleric acid and pivalic acid, ketones such as acetone, methyl ethyl ketone, methyl isobutyl ketone, diethyl ketone, dipropyl ketone, diisopropyl ketone and dibutyl ketone, and dimethylsulfoxide.

The activated catalyst selected from a palladium catalyst, a platinum catalyst, a rhodium catalyst, a ruthenium catalyst, a nickel catalyst and a cobalt catalyst in the present invention (hereinafter may be abbreviated as an "activated catalyst") means a so-called palladium catalyst, a platinum catalyst, a rhodium catalyst, a ruthenium catalyst, a nickel catalyst or a cobalt catalyst (hereinafter may be abbreviated as a "non-activated catalyst" or simply as a "catalyst") which is activated by contacting with hydrogen gas or heavy hydrogen gas.

In a method for deuteration of the present invention, deuteration may be carried out using a catalyst activated in advance, or activation of a catalyst and deuteration of a reactive substrate may be carried out simultaneously in the co-presence of a non-activated catalyst and hydrogen gas or heavy hydrogen gas in the deuteration reaction system. However, when a compound having a carbon-carbon double bond or triple bond is deuterated among the compounds represented by the general formula [1], since presence of hydrogen gas or heavy hydrogen gas in a reaction system causes hydrogenation, a catalyst activated in advance is preferably used to avoid such hydrogenation.

When deuteration is carried out using the catalyst activated by hydrogen gas or heavy hydrogen gas in advance, a part of gas phase in a deuteration reactor may be replaced with an inert gas such as nitrogen and argon.

When a deuteration reaction of the present invention is carried out in the presence of hydrogen gas or heavy hydrogen gas in a reaction system, the reaction may be carried out by directly passing hydrogen gas or heavy hydrogen gas through a reaction solution, or replacing a part of gas phase in a reactor with hydrogen gas or heavy hydrogen gas.

In the case where a compound not having a carbon-carbon double bond or triple bond is deuterated among the compounds represented by the general formula [1], deuteration reaction can be carried out by replacing a part of gas phase in a deuteration reactor with hydrogen or heavy hydrogen even if a catalyst activated in advance is used.

In a method for deuteration of the present invention, a reactor is preferably in a sealed state or nearly sealed state (hereinafter may be abbreviated as "sealed state") so that the reaction system is, as the result, in pressurized state. Nearly sealed state involves, for example, a case of so-called continuous reaction where a reactive substrate is continuously charged into a reactor and a product is continuously taken out therefrom.

In a method for deuteration of the present invention, wherein a reactor is in sealed state, temperature of a reaction system can be easily elevated to perform deuteration efficiently.

Further, by carrying out deuteration of a reactive substrate and activation of a catalyst simultaneously using a method replacing a part of gas phase in a deuteration reactor with hydrogen gas or heavy hydrogen gas, deuteration of the compound represented by the general formula [1], except one having a carbon-carbon double bond and/or triple bond, can be performed further more efficiently because such a complicated process that a catalyst is activated in advance is not required.

Furthermore, in the case where a catalyst activated by hydrogen gas or heavy hydrogen gas in advance is used in deuteration in sealed state, only deuteration proceeds without reduction, even when a substrate is a compound generally labile to reduction by hydrogen gas, and the like such as one having a carbon-carbon double bond and/or triple bond, because hydrogen gas or heavy hydrogen gas is not present in the deuteration reactor.

The activated catalyst in the present invention includes, a palladium catalyst, a platinum catalyst, a rhodium catalyst, a ruthenium catalyst, a nickel catalyst and a cobalt catalyst, as described above, and among others, a palladium catalyst, a platinum catalyst and a rhodium catalyst is preferable, a palladium catalyst and a platinum catalyst is more preferable, and a palladium catalyst is particularly preferable. These catalysts can be used effectively in a method for deuteration of the present invention by themselves or in combination accordingly.

The palladium catalyst includes one having generally 0 to 4, preferably 0 to 2 and more preferably 0 valence of a palladium atom.

The platinum catalyst includes one having generally 0 to 4, preferably 0 to 2 and more preferably 0 valence of a platinum atom.

The rhodium catalyst includes one having generally 0 or 1, preferably 0 valence of a rhodium atom.

The ruthenium catalyst includes one having generally 0 to 2, preferably 0 valence of a ruthenium atom.

The nickel catalyst includes one having generally 0 to 2, preferably 0 valence of a nickel atom.

The cobalt catalyst includes one having generally 0 or 1, preferably 0 valence of a cobalt atom.

The above catalyst may be a metal itself or oxides, halides or acetates of the above metals, and the metal catalyst may be coordinated with a ligand or may be one consisting of these metals, metal oxides, metal halides, metal acetates or metal complexes, supported on various carriers.

Hereinafter, a catalyst supported on a carrier may be abbreviated as a "carrier-supported metal catalyst", and a catalyst not supported on a carrier may be abbreviated as a "metal catalyst".

Among catalysts, in a method for deuteration of the present invention, a ligand of the metal catalyst which may be coordinated with a ligand, includes, for example, 1,5-cyclooctadiene (COD), dibenzylideneacetone (DBA), bipyridine (BPY), phenanthroline (PHE), benzonitrile (PhCN), isocyanide (RNC), triethylarsine (As(Et)₃), acetylacetone (acac); organic phosphine ligands such as dimethylphenylphosphine (P(CH₃)₂Ph), diphenylphosphinoferrocene (DPPF), trimethylphosphine (P(CH₃)₃), triethylphosphine (PEt₃), tri-tert-butylphosphine(P^{t}Bu₃), tricyclohexylphosphine (PCy₃), trimethoxyphosphine (P(OCH₃)₃), triethoxyphosphine (P(OEt)₃), tri-tert-butoxyphosphine (P(O^{t}Bu)₃), triphenylphosphine (PPh₃), 1,2-bis(diphenylphosphino)ethane (DPPE), triphenoxyphosphine (P(OPh)₃) and tri-o-tolylphosphine (P(o-tolyl)₃).

Specific examples of the palladium based metal catalyst include, for example, Pd; palladium hydroxide catalysts such as Pd(OH)₂; palladium oxide catalysts such as PdO; halogenated palladium catalysts such as PdBr₂, PdCl₂ and PdI₂; palladium acetate catalysts such as palladium acetate (Pd(OAc)₂) and palladium trifluoroacetate (Pd(OCOCF₃)₂); and palladium metal complex catalysts coordinated with a ligand such as Pd(RNC)₂Cl₂, Pd(acac)₂, diacetate-bis(triphenylphosphine)palladium [Pd(OAc)₂(PPh₃)₂], Pd(PPh₃)₄, Pd₂(dba)₃, Pd(NH₃)₂Cl₂, Pd(CH₃CN)₂Cl₂, dichlorobis(benzonitrile)palladium [Pd(PhCN)₂Cl_{2]}, Pd(dppe)Cl₂, Pd(dppf)Cl₂, Pd(PCy₃)₂Cl₂, Pd(PPh₃)₂Cl₂, Pd[P(o-tolyl)₃]₂Cl₂, Pd(cod)₂Cl₂ and Pd(PPh₃)(CH₃CN)₂Cl₂.

Specific examples of the platinum based metal catalyst include, for example, Pt; platinum catalysts such as PtO₂, PtCl₄, PtCl₂ and K₂PtCl₄; and platinum metal complex catalysts coordinated with a ligand such as PtCl₂(cod), PtCl₂(dba), PtCl₂(PCy₃)₂, PtCl₂(P(OEt)₃)₂, PtCl₂(P(O^{t}Bu)₃)₂, PtCl₂(bpy), PtCl₂(phe), Pt(PPh₃)₄, Pt(cod)₂, Pt(dba)₂, Pt(bpy)₂ and Pt(phe)2.

Specific examples of the rhodium based metal catalyst include, for example, Rh and rhodium metal complex catalysts coordinated with a ligand such as RhCl(PPh₃)₃.

Specific examples of the ruthenium based metal catalyst include, for example, Ru and ruthenium metal complex catalysts coordinated with a ligand such as RuCl₂(PPh₃)₃.

Specific examples of the nickel based metal catalyst include, for example, Ni, nickel catalysts such as NiCl₂ and NiO, and nickel metal complex catalysts coordinated with a ligand such as NiCl₂(dppe), NiCl₂(PPh₃)₂, Ni(PPh₃)₄, Ni(P(OPh)₃)₄ and Ni(cod)₂.

Specific examples of the cobalt based metal catalyst include, for example, cobalt metal complex catalysts coordinated with a ligand such as Co(C₃H₅){P(OCH₃)₃}₃.

The carrier, in the case where the above catalyst is supported on a carrier, includes, for example, carbon, alumina, silica gel, zeolite, molecular sieve, ion-exchange resins and polymers, and among others, carbon is preferable.

The ion exchange resin to be used as a carrier may be one having no adverse effect on deuteration of the present invention, and includes, for example, a cation exchange resin and an anion exchange resin.

The cation exchange resin includes, for example, a weak acidic cation exchange resin and a strong acidic cation exchange resin. The anion exchange resin includes, for example, a weak basic anion exchange resin and a strong basic anion exchange resin.

The ion exchange resin generally contains a polymer cross-linked with a difunctional monomer, as a skeleton polymer, to which an acidic group or a basic group is bonded and then is exchanged by various cations and anions (a counter ion), respectively.

Specific examples of the weak acidic cation exchange resin include, for example, one obtained by hydrolysis of a polymer of acrylate ester or a methacrylate ester, cross-linked by divinylbenzene.

Specific examples of the strong acidic cation exchange resin include, for example, one obtained by sulfonation of a copolymer of styrene-divinylbenzene.

Specific examples of the strong basic anion exchange resin include one wherein an amino group is bonded to an aromatic ring of a copolymer of stylene-divinylbenzene.

Strength of basicity of a basic anion exchange resin increases with an amino group bonded in the order of a primary amino group, a secondary amino group, a tertiary amino group and a quaternary ammonium salt.

The ion exchange resin generally available on the market may be used as well as the above ion exchange resin.

The polymer to be used as a carrier is not especially limited as long as it has no adverse effect on deuteration of the present invention, however, specific examples of such a polymer include, for example, one obtained by polymerization or copolymerization of a monomer represented by the following general formula [2]: 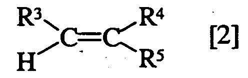 wherein R³ represents a hydrogen atom, a lower alkyl group, a carboxyl group, a carboxyalkyl group, an alkoxycarbonyl group, a hydroxyalkoxycarbonyl group, a cyano group or a formyl group; R⁴ represents a hydrogen atom, a lower alkyl group, a carboxyl group, an alkoxycarbonyl group, a hydroxyalkoxycarbonyl group, a cyano group or a halogen atom; R⁵ represents a hydrogen atom, a lower alkyl group, a haloalkyl group, a hydroxyl group, an aryl group which may have a substituent, an aliphatic heterocyclic group, an aromatic heterocyclic group, a halogen atom, an alkoxycarbonyl group, a hydroxyalkoxycarbonyl group, a sulfo group, a cyano group, a cyano-containing alkyl group, an acyloxy group, a carboxyl group, a carboxyalkyl group, an aldehyde group, an amino group, an aminoalkyl group, a carbamoyl group, a N-alkylcarbamoyl group or a hydroxyalkyl group, and R⁴ and R⁵ may form an alicyclic ring together with the adjacent -C=C- bond.

In the general formula [2], the lower alkyl group represented by R³ to R⁵ may be straight chained, branched or cyclic, and includes, for example, an alkyl group having 1 to 6 carbon atoms, which is specifically exemplified by a methyl group, an ethyl group, a n-propyl group, an isopropyl group, a n-butyl group, an isobutyl group, a tert-butyl group, a sec-butyl group, a n-pentyl group, an isopentyl group, a tert-pentyl group, a 1-methylpentyl group, a n-hexyl group, an isohexyl group, a cyclopropyl group, a cyclopentyl group and a cyclohexyl group.

The carboxyalkyl group represented by R³ and R⁴ includes, for example, one wherein a part of hydrogen atoms of the above lower alkyl group is replaced by a carboxyl group, which is specifically exemplified by, for example, a carboxymethyl group, a carboxyethyl group, a carboxypropyl group, a carboxybutyl group, a carboxypentyl group and a carboxyhexyl group.

The alkoxycarbonyl group represented by R³ to R⁵ includes, for example, preferably one having 2 to 11 carbon atoms, which is specifically exemplified by, for example, a methoxycarbonyl group, an ethoxycarbonyl group, a propoxycarbonyl group, a butoxycarbonyl group, a pentyloxycarbonyl group, a hexyloxycarbonyl group, a heptyloxycarbonyl group, a 2-ethylhexyloxycarbonyl group, an octyloxycarbonyl group, a nonyloxycarbonyl group and a decyloxycarbonyl group.

The hydroxyalkoxycarbonyl group represented by R³ to R⁵ includes one wherein a part of hydrogen atoms of the above alkoxycarbonyl group having 2 to 11 carbon atoms is replaced by a hydroxyl group, which is specifically exemplified by, for example, a hydroxymethyloxycarbonyl group, a hydroxyethyloxycarbonyl group, a hydroxypropyloxycarbonyl group, a hydroxybutyloxycarbonyl group, a hydroxypentyloxycarbonyl group, a hydroxyhexyloxycarbonyl group, a hydroxyheptyloxycarbonyl group, a hydroxyoctyloxycarbonyl group, a hydroxynonyloxycarbonyl group and a hydroxydecyloxycarbonyl group.

The halogen atom represented by R⁴ and R⁵ includes, for example, fluorine, chlorine, bromine and iodine.

The haloalkyl group represented by R⁵ includes, for example, one having 1 to 6 carbon atoms, wherein the above lower alkyl group represented by R³ to R⁵ is halogenated (for example, fluorinated, chlorinated, brominated, iodinated, etc.), which is specifically exemplified by, for example, a chloromethyl group, a bromomethyl group, a trifluoromethyl group, a 2-chloroethyl group, a 3-chloropropyl group, a 3-bromopropyl group, a 3,3,3-trifluoropropyl group, a 4-chlorobutyl group, a 5-chloropentyl group and a 6-chlorohexyl group.

The aryl group of the aryl group which may have a substituent includes, for example, a phenyl group, a tolyl group, a xylyl group and a naphthyl group, and said substituent includes, for example, an amino group, a hydroxyl group, a lower alkoxy group and a carboxyl group. Specific examples of the substituted aryl group include, for example, an aminophenyl group, a toluidino group, a hydroxyphenyl group, a methoxyphenyl group, a tert-butoxyphenyl group and a carboxyphenyl group.

The aliphatic heterocyclic group includes, for example, preferably a 5- or 6-membered one having 1 to 3 hetero atoms such as a nitrogen atom, an oxygen atom and a sulfur atom, which is specifically exemplified by, for example, a 2-oxopyrrolidyl group, a piperidyl group, a piperidino group, a piperazinyl group and a morpholino group.

The aromatic heterocyclic group includes, for example, preferably a 5- or 6-membered one having 1 to 3 hetero atoms such as a nitrogen atom, an oxygen atom and a sulfur atom, which is specifically exemplified by; for example, a pyridyl group, an imidazolyl group, a thiazolyl group, a furyl group and a pyranyl group.

The cyano-containing alkyl group includes, for example, one wherein a part of hydrogen atoms of the above lower alkyl group is replaced by a cyano group, which is specifically exemplified by, for example, a cyanomethyl group, a 2-cyanoethyl group, a 2-cyanopropyl group, a 3-cyanopropyl group, a 2-cyanobutyl group, a 4-cyanobutyl group, a 5-cyanopentyl group and a 6-cyanohexyl group.

The acyloxy group includes, for example, one derived from a carboxylic acid having 2 to 20 carbon atoms, which is specifically exemplified by, for example, an acetyloxy group, a propionyloxy group, a butyryloxy group, a pentanoyloxy group, a nonanoyloxy group, a decanoyloxy group and a benzoyloxy group.

The aminoalkyl group includes one wherein a part of hydrogen atoms of the above lower alkyl group is replaced by an amino group, which is specifically exemplified by, for example, an aminomethyl group, an aminoethyl group, an aminopropyl group, an aminombutyl group, an aminopentyl group and an aminohexyl group.

The N-alkylcarbamoyl group includes one wherein a part of hydrogen atoms of a carbamoyl group is replaced by an alkyl group, which is specifically exemplified by, for example, an N-methylcarbamoyl group, an N-ethylcarbamoyl group, an N-n-propylcarbamoyl group, an N-isopropylcarbamoyl group, an N-n-butylcarbamoyl group and an N-tert-butylcarbamoyl group.

The hydroxyalkyl group includes one wherein a part of hydrogen atoms of the above lower alkyl group is replaced by a hydroxyl group, which is specifically exemplified by, for example, a hydroxymethyl group, a hydroxyethyl group, a hydroxypropyl group, a hydroxybutyl group, a hydroxypentyl group and a hydroxyhexyl group.

The aliphatic ring in the case where R⁴ and R⁵ are bonded together with the adjacent -C=C- bond to form an alicyclic ring, includes, for example, an unsaturated alicyclic ring having 5 to 10 carbon atoms, and may be monocyclic or polycyclic, which is specifically exemplified by, for example, a norbornene ring, a cyclopentene ring, a cyclohexene ring, a cyclooctene ring and a cyclodecene ring.

The specific examples of the monomer represented by the general formula [2] include, for example, ethylenically unsaturated aliphatic hydrocarbons having 2 to 20 carbon atoms such as ethylene, propylene, butylene and isobutylene; ethylenically unsaturated aromatic hydrocarbons having 8 to 20 carbon atoms such as styrene, 4-methylstyrene, 4-ethylstyrene and divinylbenzene; alkenyl esters having 3 to 20 carbon atoms such as vinyl formate, vinyl acetate, vinyl propionate and isopropenyl acetate; halogen-containing ethylenically unsaturated compounds having 2 to 20 carbon atoms such as vinyl chloride, vinylidene chloride, vinylidene fluoride and tetrafluoroethylene; ethylenically unsaturated carboxylic acids having 3 to 20 carbon atoms such as acrylic acid, methacrylic acid, itaconic acid, maleic acid, fumaric acid, crotonic acid, vinylacetic acid, allylacetic acid and vinylbenzoic acid (these acids may form a salt of alkali metals such as sodium and potassium, or an ammonium salt); ethylenically unsaturated carboxylic acid esters such as methyl methacrylate, ethyl methacrylate, propyl methacrylate, butyl methacrylate, 2-ethylhexyl methacrylate, methyl acrylate, ethyl acrylate, propyl acrylate, butyl acrylate, 2-ethylhexyl acrylate, lauryl methacrylate, stearyl acrylate, methyl itaconate, ethyl itaconate, methyl maleate, ethyl maleate, methyl fumarate, ethyl fumarate, methyl crotonate, ethyl crotonate and methyl 3-butenoate; cyano-containing ethylenically unsaturated compounds having 3 to 20 carbon atoms such as acrylonitrile, methacrylonitrile and allyl cyanide; ethylenically unsaturated amide compounds having 3 to 20 carbon atoms such as acrylamide and methacrylamide; ethylenically unsaturated aldehydes having 3 to 20 carbon atoms such as acrolein and crotonaldehyde; ethylenically unsaturated sulfonic acids having 2 to 20 carbon atoms such as vinylsulfonic acid and 4-vinylbenzene sulfonic acid (these acids may form a salt of alkali metals such as sodium and potassium); ethylenically unsaturated aliphatic amines having 2 to 20 carbon atoms such as vinylamine and allylamine; ethylenically unsaturated aromatic amines having 8 to 20 carbon atoms such as vinylaniline; ethylenically unsaturated aliphatic heterocyclic amines having 5 to 20 carbon atoms such as N-vinylpyrrolidone and vinylpiperidine; ethylenically unsaturated alcohols having 3 to 20 carbon atoms such as allyl alcohol and crotyl alcohol; and ethylenically unsaturated phenols having 8 to 20 carbon atoms such as 4-vinylphenol.

When the above polymer is used as a carrier, use of the carrier that is hardly deuterated itself by deuteration of the present invention is preferable, however, a catalyst supported on the carrier deuteratable itself can be also used for deuteration of the present invention.

In a method for deuteration of the present invention, among the catalysts supported on a carrier, a carrier-supported palladium catalyst, a carrier-supported platinum catalyst and a carrier-supported rhodium catalyst are preferably used. Among others, a carrier-supported palladium catalyst is preferable and specifically a palladium carbon is particularly preferable.

In the carrier-supported catalysts, a ratio of palladium, platinum, rhodium, ruthenium, nickel or cobalt, as a catalyst metal is generally 1 to 99% by weight, preferably 1 to 50% by weight, more preferably 1 to 30% by weight, further more preferably 1 to 20% by weight and particularly preferably 5 to 10% by weight, based on the whole catalyst.

In a method for deuteration of the present invention, an amount of the activated catalyst or non-activated catalyst to be used is generally so-called catalyst quantity, and preferably in the order 0.01 to 200% by weight, 0.01 to 100% by weight, 0.01 to 50% by weight, 0.01 to 20% by weight, 0.1 to 20% by weight, 1 to 20% by weight and 10 to 20% by weight, relative to the compound represented by the general formula [1] to be used as a reactive substrate, irrespective of whether the catalyst is supported on a carrier or not, and the upper limit content of the catalyst metal in said whole catalyst is preferably in the order of, 20% by weight, 10% by weight, 5% by weight and 2% by weight, while the lower limit thereof is preferably in the order of, 0.0005% by weight, 0.005% by weight, 0.05% by weight and 0.5% by weight.

When a compound represented by the above general formula [1] is deuterated, two or more kinds of the various catalysts as described above can be used in an appropriate combination as a catalyst. Such combined use of the catalysts can sometimes improve deuteration ratio. For example, in the case where a compound represented by the general formula [1], wherein X is a hydroxymethylene group, is deuterated, specific examples of the combination of catalysts to improve deuteration ratio include, for example, a combination of a palladium catalyst and a platinum catalyst, a ruthenium catalyst or a rhodium catalyst, a combination of a platinum catalyst and a ruthenium catalyst or a rhodium catalyst, and a combination of a ruthenium catalyst and a rhodium catalyst, and among others, preferably a combination of a palladium catalyst and a platinum catalyst, wherein any one or both thereof may be supported on a carrier. Specific examples of the combination of a palladium catalyst and a platinum catalyst include, for example, a combination of a palladium carbon and a platinum carbon.

In the case where two or more kinds of catalysts are used in combination, amounts of the catalysts to be used may be determined so that the total amount of the catalysts becomes the amount of the catalyst to be used as described above. an amount ratio of each catalyst to be used is not especially limited, however, when a palladium carbon and a platinum carbon are used in combination as described above, an amount of each catalyst to be used may be determined so that weight of palladium in the whole catalyst becomes generally 0.01 to 100 times, preferably 0.1 to 10 times and more preferably 0.2 to 5 times, relative to weight of platinum.

In the case where a non-activated catalyst is used in the reaction of the present invention, an amount of hydrogen to be used, wherein hydrogen is present in the reaction system to activate the catalyst, may be a necessary amount to activate the catalyst, and the amount is generally 1 to 20,000 equivalents and preferably 10 to 700 equivalents, relative to the catalyst, because these is possibility that an excessive amount of hydrogen shows adverse effect on a deuteration reaction of the present invention, such as hydrogenation of a deuterated solvent as a heavy hydrogen source and decrease of a ratio of heavy hydrogen as a heavy hydrogen source in the reaction system.

Further, in the case where heavy hydrogen is present in the reaction system to activate the catalyst, an amount of heavy hydrogen to be used may be a necessary amount to activate the catalyst, and the amount is generally 1 to 20,000 equivalents and preferably 10 to 700 equivalents, relative to the catalyst, however, even if the amount of said heavy hydrogen is excessively large, deuteration of the present invention can be performed without any problem, because said heavy hydrogen can be also used as a heavy hydrogen source of the present invention.

In a method for deuteration of the present invention, the lower limit of reaction temperature is generally 10°C, and preferably in order of, 20°C, 40°C, 60°C, 80°C, 110°C, 140°C and 160°C, and the upper limit thereof is generally 300°C, and preferably in order of 200°C and 180°C.

In a method for deuteration of the present invention, a reaction time is generally 30 minutes to 72 hours, preferably 1 to 48 hours, more preferably 3 to 30 hours, and further more preferably 6 to 24 hours.

The method for deuteration of the present invention will be specifically explained by taking, as an example, the case of using heavy water as a heavy hydrogen source and a palladium carbon catalyst (Pd/ C) (Pd content: 10%) as a non-activated catalyst.

Namely, for example, 1 mole of a compound represented by the general formula [1] not having a carbon-carbon double bond nor a carbon-carbon triple bond in structure thereof (substrate) and 0.01 to 200% by weight of a non-activated Pd/C relative to said substrate are added to such amount of heavy water that 10 to 150 molar times of heavy hydrogen atoms relative to deuteratable hydrogen atoms in the substrate are contained therein, followed by replacing atmosphere of a sealed reaction system by hydrogen and reacting, with stirring in an oil bath at about 110°C to 200°C for about 1 to 48 hours. After completion of the reaction, when the reaction product is soluble in a deuterated solvent, the catalyst is filtered off from the reaction solution, and the filtrate is concentrated to isolate the product which is subjected to structural analysis by ¹H-NMR, ²H-NMR and mass spectrum measurements.

When the reaction product is hardly soluble in the deuterated solvent, the reaction product is isolated from the reaction solution and then subjected to structural analysis by ¹H-NMR, ²H-NMR and mass spectrum measurements. In the case where isolation of the reaction product from the reaction solution is difficult, the filtrate as it is may be subjected to measurement of ¹H-NMR to perform structural analysis by using an appropriate internal standard substance.

When the product is hardly soluble in a deuterated solvent, the isolation of the product from the reaction solution may be carried out according to known purification methods such as extraction of the product from the reaction solution using an organic solvent, in which the product is soluble and then filtering off the catalyst.

The method for deuteration of the present invention will further be explained specifically by taking, as another example, the case of using heavy water as a heavy hydrogen source and a palladium carbon catalyst (Pd content: 10%) activated by hydrogen gas as a catalyst activated in advance.

Namely, for example, 1 mole of a compound represented by the general formula [1] having a carbon-carbon double bond or a carbon-carbon triple bond in structure thereof (substrate) and 0.01 to 200% by weight of a Pd/C catalyst, relative to said substrate, activated by contacting with hydrogen gas in advance are added to such amount of heavy water that 10 to 150 molar times of heavy hydrogen atoms relative to deuteratable hydrogen atoms in the substrate are contained therein, followed by replacing atmosphere of a sealed reaction system with inert gas and reacting with stirring in an oil bath at about 110°C to 200°C for about 1 to 48 hours. After completion of the reaction, when the reaction product is soluble in a deuterated solvent, the catalyst is filtered out from the reaction solution, and the filtrate is concentrated to isolate the product which is subjected to structural analysis by ¹H-NMR, ²H-NMR and mass spectrum measurements.

When the reaction product is hardly soluble in the deuterated solvent, the reaction product is isolated from the reaction solution and then subjected to structural analysis by ¹H-NMR, ²H-NMR and mass spectrum measurements. In the case where isolation of the reaction product from the reaction solution is difficult, the filtrate as it is may be subjected to measurement of ¹H-NMR to perform structural analysis by using an appropriate internal standard substance. Isolation of the product from the reaction solution may be carried out in the same manner as of the isolation method In a method for deuteration of the present invention using a non-activated catalyst. Further, when the product is hardly soluble in a deuterated solvent, isolation of the product from the reaction solution may be carried out according to known purification methods such as extraction of the product from the reaction solution using an organic solvent and the like, in which the product is soluble and then filtering off the catalyst.

Further, among the methods for deuteration of the present invention, for example, by the method using a palladium carbon catalyst and a platinum carbon catalyst in combination, tricyclo[5.2.1.0^{2,6}]decan-8-ol, wherein deuteration ratio thereof is generally not less than 60%, preferably in order of, not less than 70%, not less than 78%, not less than 80%, not less than 85%, not less than 88%, not less than 89% and not less than 90%, can be easily obtained. Thus obtained deuterated tricyclo[5.2.1.0^{2,6}]decan-8-ol is a very useful compound, for example, as a raw material of deuterated methacrylate ester for a polymer of optical fiber.

As described above, the method for deuteration of the present invention using a catalyst activated in advance as an activated catalyst and a deuterated solvent as a heavy hydrogen source, can perform only the desired deuteration, even when a compound represented by the general formula [1] has a carbon-carbon double bond or a carbon-carbon triple bond, without reduction of the double or triple bond by hydrogenation, and even when said compound has a substituent such as a nitro group and a cyano group, without reduction of these substituents.

When a compound represented by the general formula [1] has a carbon-carbon double bond or triple bond, wherein these bonds tend to be easily polymerized during the deuteration reaction of the present invention, for example, a polymerization inhibitor, and the like may be added to the reaction system of the deuteration reaction to inhibit the polymerization reaction.

As described above, the method for deuteration of the present invention, comprising reacting a compound represented by the general formula [1] with a heavy hydrogen source in the co-presence of an activated catalyst, enables to efficiently carry out the deuteration (replacement by deuterium or tritium) of a compound having a carbonyl group and a secondary alcohol compound, irrespective of presence or absence of a double bond or triple bond in the compound or presence or absence of a substituent and a type thereof.

Further, the method for deuteration of the present invention can not only improve a working environment but also be applied to deuteration of a substrate which is labile to decomposition at high temperature or under acidic or basic condition, because the deuteration reaction can be carried out without making the reaction condition particularly acidic or basic.

Furthermore, in a compound represented by the general formula [1] wherein X is a carbonyl group, the method for deuteration of the present invention enables to efficiently deuterate not only a hydrogen atom locating near the carbonyl group but also a hydrogen atom locating far from the carbonyl group.

Further, in a compound represented by the general formula [1] wherein X is a hydroxymethylene group, the method for deuteration of the present invention enables to efficiently deuterate not only a hydrogen atom locating far from the hydroxyl group but also a hydrogen atom locating near the hydroxyl group.

Still further, among the compounds represented by the general formula [1], particularly in tricyclo[5.2.1.0^{2,6}]decan-8-ol, the method for deuteration of the present invention enables to obtain one wherein a deuteration ratio thereof is higher than the level that can be achieved by conventional methods.

In the following, the present invention will be explained in further detail referring to Examples, but the present invention is not limited thereto by any means.

In the Examples, following catalysts were used: palladium carbon (Pd/C) with Pd content of 10%, platinum carbon (Pt/C) with Pt content of 5%, ruthenium carbon (Ru/C) with Ru content of 5% and rhodium carbon (Rh/C) with Rh content of 5%.

### EXAMPLE

### Example 1

In 17 mL of deuterium oxide (D₂O) were suspended 500 mg of 4-heptanone (substance) and 50 mg of palladium carbon, followed by replacing atmosphere of a sealed reaction system with hydrogen gas and conducting a reaction in an oil bath at 160°C for about 24 hours. After completion of the reaction, the reaction solution was extracted with ether, followed by filtering off the catalyst from the obtained extract and concentration of the filtrate under reduced pressure to' obtain a product, which was subjected to structural analysis by ¹H-NMR, ²H-NMR and mass spectrum measurements. Isolation yield of the objective product was 46% and deuteration ratio of the substrate was 97%.

### Example 2

In 17 mL of deuterium oxide were suspended 500 mg of acetone (substrate) and 50 mg of palladium carbon, followed by replacing the atmosphere of a reaction system with hydrogen gas and conducting a reaction in an oil bath at 110°C for about 24 hours. After completion of the reaction, the catalyst was filtered off from the reaction solution. In the filtrate was added dioxane as an internal standard and then subjected to structural analysis by ¹H-NMR measurement. Deuteration ratio of the substrate was 99%.

### Examples 3 to 15

The same deuteration reactions as in Example 1 were conducted, except for using substrates as deuteration targets and catalysts shown in the following Table 1 and conducting at each temperature shown in Table 1. Isolation yields and deuteration ratios of the obtained compounds are shown in Table 1. In Table 1, deuteration ratios of 2-butanone, 2-norbornanone, tricyclo[5.2.1.0^{2,6}]decane-8-one, norborneol, tricyclo[5.2.1.0^{2,6}]-3-decen-8-ol (hydroxydicyclopentadiene) and cyclohexanol mean deuteration ratios at the positions denoted by the numerals in each of the following chemical formulas. Further, in Table 1, the isolation yields denoted by "―" mean that deuteration ratios were measured without isolating objective products after the deuteration.

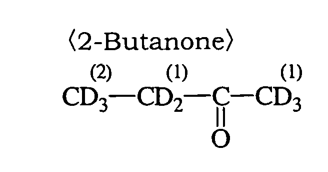

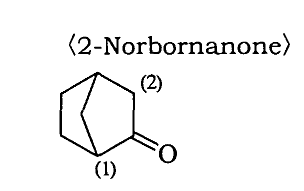

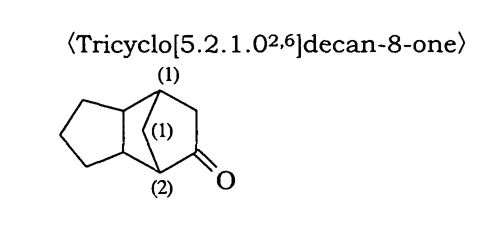

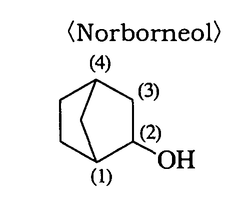

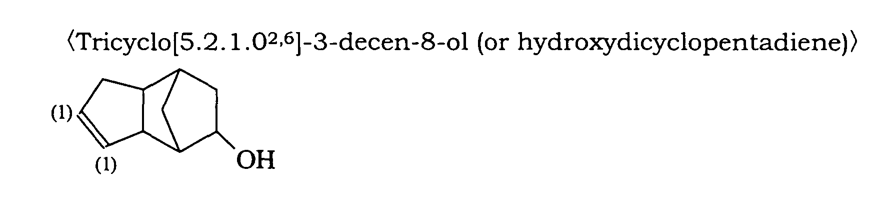

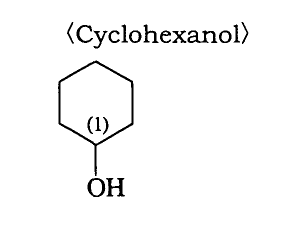

**Table 1**

| | Substrate | Catalyst | Reaction Temperature | Isolation Yield (%) | Deuteration Ratio (%) |
|---|---|---|---|---|---|
| Example 3 | 2-Butanone | Pd/C | 110°C | - | (1) 92, (2) 80 |
| Example 4 | 2-Heptanone | Pd/C | 160°C | 30 | 97 |
| Example 5 | 3-Heptanone | Pd/C | 160°C | 32 | 97 |
| Example 6 | Cyclohexanone | Pd/C | 180°C | 40 | 95 |
| Example 7 | 2-Norbornanone | Pd/C | 180°C | 90 | (1) 43, (2) 99, Others 27 |
| Example 8 | Trir-yclo[5.2.1.0^{2,6}] decan-8-one | Pd/C | 180°C | 88 | (1) 20, (2) 40, (3) 99, Others 10 |
| Example 9 | Sodium acetate | Pd/C | 160°C | 100 | 50 |
| Example 10 | Isobutylic acid | Pd/C | 160°C | - | 40 |
| Example 11 | 2-Heptanol | Pd/C | 160°C | 44 | 96 |
| Example 12 | 4-Heptanol | Pd/C | 160°C | 32 | 87 |
| Example 13 | Norborneol | Pd/C | 180°C | 32 | (1) 38, (2) 72, (3) 66, (4) 28, Others 47 |
| Example 14 | Tricydo[5.2.1.0^{2,6}] -3-decen-8-ol | Pd/C | 180°C | 86 | (1) 50, Others 25 |
| Example 15 | Cyclohexanol | Pd/C | 180°C | 66 | (1) 67, Others 77 |

### Example 16

In 17 mL of deuterium oxide were suspended 500 mg of tricyclo[5.2.1.0^{2,6}]decan-8-ol (substrate) and 50 mg of palladium carbon, followed by replacing atmosphere of a sealed reaction system with hydrogen gas and conducting a reaction in an oil bath at 180°C for about 24 hours. After completion of the reaction, the reaction solution was extracted with ether, followed by filtering off the catalyst and concentration of the filtrate under reduced pressure to obtain a product, which was subjected to structural analysis by ¹H-NMR, ²H-NMR and mass spectrum measurements. Isolation yield and deuteration ratio of the objective product was 60% and 45%, respectively. Results are shown in Table 2. In Table 2, amount of metal (% by weight) means amount ratio of catalyst metal present in a carrier-supported catalyst relative to amount of the substrate, and deuteration ratio in Table 2 means an average deuteration ratio of the whole deuteratable hydrogen atoms, and provided that (1) means deuteration ratio at the position denoted by (1) in the following chemical formula and "others" means average deuteration ratio at the other positions than (1).

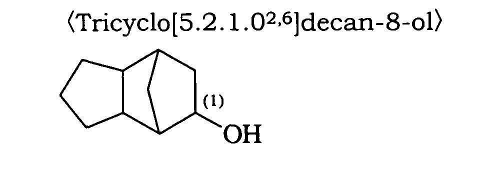

### Examples 17 to 26

Deuteration of tricyclo[5.2.1.0^{2,6}]decan-8-ol was carried out in the same manner as in Example 16 except for using catalysts shown in Table 2 in amounts shown in Table 2 and reacting for reaction times shown in Table 2. Results are shown together in Table 2.

**Table 2**

| | Catalyst and Amount thereof | Amount of Metal | Reaction Time | Isolation Yield (%) | Deuteration Ratio (%) |
|---|---|---|---|---|---|
| Example 16 | Pd/C, 50mg | 1% by weight | 24 hours | 60 | 45 |
| Example 17 | Pt/C, 100mg | 1% by weight | 24 hours | 36 | 61 |
| Example 18 | Pt/C, 100mg | 1% by weight | 48 hours | 36 | 74 |
| Example 19 | Ru/C, 100mg | 1% by weight | 24 hours | 53 | (1) 100, Others 15 |
| Example 20 | Rh/C,100mg | 1% by weight | 24 hours | 58 | (1) 51, Others 32 |
| Example 21 | Pd/C, 200mg | 4% by weight | 24 hours | 40 | 70 |
| Example 22 | Pd/C, 250md | 5% by weight | 24 hours | 50 | 75 |
| Example 23 | Pd/C, 250mg | 5% by weight | 48 hours | 23 | 87 |
| Example 24 | Pd/C, 50mg Pt/C, 100mg | 2% by weight | 24 hours | 44 | 78 |
| Example 25 | Pd/C, 100mg Pt/C, 200mg | 4% by weight | 24 hours | 41 | (1) 96, Others 88 |

### Example 26

In 17 mL of deuterium oxide, was suspended palladium carbon, followed by replacing atmosphere of a reaction system with a hydrogen gas and then stirring at room temperature for 3 hours to activate the palladium carbon. After completion of the activation, 500 mg of sodium methacrylate (substrate) was charged thereto, followed by replacing atmosphere of the reaction system with nitrogen gas and conducting a reaction in an oil bath at 180°C for about 24 hours. After completion of the reaction, the reaction solution was filtered to remove the catalyst, followed by concentration under reduced pressure to obtain a compound, which was subjected to structural analysis by ¹H-NMR and ²H-NMR measurements. Isolation yield and deuteration ratio of the substrate were 100% and not less than 99%, respectively.

### Examples 27 to 32

The same deuteration reactions as in Example 26 were conducted, except for using substrates as deuteration targets and catalysts, shown in the following Table 3 and conducting at each reaction temperature shown in Table 3. Isolation yields and deuteration ratios of thus obtained compounds are shown together in Table 3. In Table 3, isolation yield denoted by "-" means the same as in Table 1.

**Table 3**

| | Substrate | Catalyst | Reaction Temperature | Isolation Yield (%) | Deuteration Ratio (%) |
|---|---|---|---|---|---|
| Example 26 | Sodium methacrylate | Pd/C | 180°C | 100 | 99≦ |
| Example 27 | Sodium methacrylate | Rh/C | 160°C | 100 | 98 |
| Example 28 | Sodium methacrylate | Rh/alumina | 160°C | 100 | 98 |
| Example 29 | Sodium methacrylate | Pt/C | 180°C | 100 | 75 |
| Example 30 | Sodium methacrylate | Raney-Ni | 160°C | 100 | 52 |
| Example 31 | Sodium methacrylate | Ru/C | 160°C | 100 | 23 |
| Example 32 | Methacrylic acid | Pd/C | 180°C | - | 90 |

### Comparative Example 1

The same deuteration reaction as in Example 26 was conducted, except for using methacrylic acid as a substrate and non-activated palladium carbon as a catalyst, and the obtained compound was subjected to structural analysis by ¹H-NMR, ²H-NMR measurements. Deuteration ratio of the substrate was 75%.

### Comparative Example 2

The same deuteration reaction as in Example 26 was conducted, except for using methacrylic acid as a substrate and heavy hydrogen gas as a heavy hydrogen source, and the obtained compound was subjected to structural analysis by ¹H-NMR, ²H-NMR measurements. It was confirmed that the carbon-carbon double bond of methacrylic acid was reduced, though deuteration was performed.

It is clear from the results of Examples 1 to 32 that a compound having a carbonyl group or a hydroxyl group can be efficiently deuterated by the method for deuteration of the present invention.

It is also clear from the results of Examples 1 to 25 that in the case where a compound not having a carbon-carbon double bond is deuterated, activation of a catalyst and a deuteration reaction can be efficiently carried out simultaneously in a reaction system.

From the results of Examples 24 and 25, it is clear that deuteration can be performed by using two or more kinds of catalysts in combination.

From the results of Example 24 wherein palladium carbon and platinum carbon were used in combination, though total amount of the catalyst metals was as low as 2% by weight relative to a substrate, it is clear that obviously higher deuteration ratio can be obtained in comparisons with Examples 21 and 22 wherein a single catalyst of palladium carbon was used and amount of the catalyst metal was comparatively as high as 4% by weight or 5% by weight.

From comparison of the results of Examples 21 and 25, it is clear that Example 25, wherein deuteration was carried out by using palladium carbon and platinum carbon in combination, can provide higher deuteration ratio in comparison with Example 21 wherein deuteration was carried out using only palladium carbon as a catalyst, though amount of the catalyst metal(s) in each Example was the same (4% by weight).

Further, from comparisons of the results of Examples 26 to 32 and Comparative Example 2, it is clear that even when a carbonyl compound or a secondary alcohol containing a carbon-carbon double bond or triple bond is deuterated, only objective deuteration proceeds without reduction of said double or triple bond by the method for deuteration of the present invention.

Further, from comparison of the results of Example 32 and Comparative Example 1, it is clear that the method for deuteration of the present invention, wherein an activated catalyst is used, gives higher deuteration ratio in comparison with the case wherein a non-activated catalyst is used.

Still further, from the results of Examples 1 to 32, it is clear that the method for deuteration of the present invention can efficiently deuterate without making a reaction solution under basic condition.

### INDUSTRIAL APPLICABILITY

A method for deuteration (replacement by deuterium or tritium) of the present invention, which comprises reacting a compound represented by the general formula [1] with a heavy hydrogen source in the co-presence of an activated catalyst can significantly improve working environment, because the deuteration which has been conventionally carried out under severe conditions such as basic condition can be carried out under neutral condition. Further, even when the compound represented by the general formula [1] is one having a carbon-carbon double bond or triple bond, the method for deuteration of the present invention enables to efficiently carry out objective deuteration without reduction of said double bond or triple bond.

## Claims

1. A method for deuteration of a compound represented by the general formula [1]:
R¹―X―R² [1]
wherein, R¹ represents an alkyl group or an aralkyl group, which may have a carbon-carbon double bond and/or triple bond; R² represents an alkyl group which may have a carbon-carbon double bond and/or triple bond, an aryl group, an aralkyl group, an alkoxy group, an aryloxy group or a hydroxyl group; X represents a carbonyl group or a hydroxylmethylene group; R¹ and R² may form an alicyclic ring together with a carbon atom contained in X; provided that R² represents an alkyl group which may have a carbon-carbon double bond and/or triple bond, an aryl group or an aralkyl group when X is a hydroxylmethylene group,
comprising reacting the compound represented by the general formula [1] with a heavy hydrogen source in the co-presence of an activated catalyst selected from a palladium catalyst, a platinum catalyst, a rhodium catalyst, a ruthenium catalyst, a nickel catalyst and a cobalt catalyst.

2. The method for deuteration according to claim 1, wherein X is a carbonyl group in the general formula [1].

3. The method for deuteration according to claim 1, wherein X is a hydroxymethylene group in the general formula [1].

4. The method for deuteration according to any one of claims 1 to 3, wherein the heavy hydrogen source is a deuterated solvent.

5. The method for deuteration according to claim 4, wherein the deuterated solvent is deuterium oxide (D₂O).

6. The method for deuteration according to any one of claims 1 to 5, wherein the activated catalyst selected from a palladium catalyst, a platinum catalyst, a rhodium catalyst, a ruthenium catalyst, a nickel catalyst and a cobalt catalyst is one obtained by activating a non-activated catalyst selected from a palladium catalyst, a platinum catalyst, a rhodium catalyst, a ruthenium catalyst, a nickel catalyst and a cobalt catalyst by contacting with hydrogen gas or heavy hydrogen gas.

7. The method for deuteration according to claim 6, wherein the contact of a non-activated catalyst selected from a palladium catalyst, a platinum catalyst, a rhodium catalyst, a ruthenium catalyst, a nickel catalyst and a cobalt catalyst with hydrogen gas or heavy hydrogen gas is conducted in a deuteration reaction system.

8. The method for deuteration according to any one of claims 1 to 7, wherein the activated catalyst selected from a palladium catalyst, a platinum catalyst, a rhodium catalyst, a ruthenium catalyst, a nickel catalyst and a cobalt catalyst is a catalyst comprising an activated palladium based catalyst.

9. The method for deuteration according to claim 8, wherein the activated palladium based catalyst is an activated palladium carbon.

10. The method for deuteration according to claim 8, wherein the catalyst comprising an activated palladium based catalyst is a catalyst comprising an activated palladium catalyst and an activated platinum catalyst.

11. The method for deuteration according to claim 1, wherein the compound represented by the general formula [1] is tricyclo[5.2.1.0^{2,6}]decan-8-ol, and the activated catalyst selected from a palladium catalyst, a platinum catalyst, a rhodium catalyst, a ruthenium catalyst, a nickel catalyst and a cobalt catalyst is a catalyst comprising palladium carbon and platinum carbon.

12. Tricyclo[5.2.1.0^{2,6}]decan-8-ol wherein deuteration ratio thereof is 60% or more.
